**Europäisches Patentamt**

(19) **European Patent Office**

**Office européen des brevets**

(11) Publication number: **0 173 161**
**B1**

(12) **EUROPEAN PATENT SPECIFICATION**

(45) Date of publication of patent specification: **31.01.90**

(21) Application number: **85110119.6**

(22) Date of filing: **12.08.85**

(51) Int. Cl.⁵: **A 61 F 5/00, A 61 F 5/04**

(54) Multiaxis controlled motion knee orthosis.

(30) Priority: **13.08.84 US 639866**

(43) Date of publication of application:
**05.03.86 Bulletin 86/10**

(45) Publication of the grant of the patent:
**31.01.90 Bulletin 90/05**

(84) Designated Contracting States:
**AT CH DE FR GB IT LI SE**

(56) References cited:
**WO-A-84/04240**
**US-A-2 883 982**
**US-A-3 552 786**
**US-A-4 353 361**
**US-A-4 370 977**

(73) Proprietor: **TOWNSEND INDUSTRIES, INC.**
**4630 Easton Drive**
**Bakersfield, California 93309 (US)**

(72) Inventor: **Townsend, Jeffrey H.**
**7631 Parkway**
**Bakersfield, California 93304 (US)**

(74) Representative: **WILHELMS, KILIAN & PARTNER**
**Patentanwälte**
**Eduard-Schmid-Strasse 2**
**D-8000 München 90 (DE)**

## Description

The present invention relates to orthopaedic devices for the stabilization and control of a human knee joint along several axes and has as its primary purpose the stabilization of the knee joint which has been injured. Stabilization and control is achieved in such a manner as to permit the user relative freedom in the normal use of the bones while at the same time, permitting control over the joint so as to optimize healing.

The human knee is acknowledged as one of the weakest joints in the body. It is the articulating joint between the thigh and calf muscle groups which support the body's weight while walking or running, and it is held together Primarily by two small but powerful ligaments, the anterior and posterior cruciate ligaments. Knee instability arising out of cartilage damage, ligament strain and other such causes is relatively commonplace since the knee joint is subjected to significant loads during the course of almost any kind of physical activity requiring the use of the legs. Devices for adding support and strength to the knee joint have been known at least since the very early Sears and Roebuck catalogs, which provided elasticized support members intended to circumscribe the joint to apply a squeezing pressure which results in a "feeling" of additional support even though that support may be phyiologically minimal.

While the public has been sports-minded for years, it was not until the late 1960's, with a great increase in the volume of televised professional sports, and very highly paid athletes, and, in particular, contact sports such as football, basketball and soccer, that sports medicine, as a specialized field, evolved. Until that time, knee braces typically consisted of two sets of links connected by a pivot pin which were disposed on either side of the knee and sewn into an elasticized sleeve which was slipped over the knee joint like an elastic sock so that the pivot pins were disposed on either side of the knee. Such devices afforded virtually no protection to an athlete engaged in a contact sport, or in a sport where there is a large amount of stress placed on the knee joint due to running, jumping, pivoting and the like.

An appliance according to the preamble of claim 1 is known from US-A-4 353 361. In this known appliance, each femoral link is connected to an intermediate member through a pivot pin, this intermediate member being connected through a second pivot pin which is in parallel to the first-mentioned pivot pin to the associated tibial link. The joint designed in this way allows a tibial link to perform a sliding and rotational motion in respect of its associated femoral link, the joint thus being able to mimic the motions of an associated anatomical joint. This known joint exhibits no predetermined pattern of sliding and rotational motions between terminal points, but can be moved along a varity of different paths.

US-A-3 552 786 describes a knee joint comprising camming means which allow the joint to perform a sliding movement between femoral and tibial members during an initial range of flexion and to rotate thereafter along a predetermined arcuate path. However, there is a deliberately induced play between a displaceable cam member and a camming slot in order to avoid catches or hangups during the sliding motion. An elastomeric compression member is provided to compensate for any play or looseness in the joint assembly. However, the biasing effect of the elastomeric compression member can be overcome during use so that a proper guidance of a malfunctioning knee of the wearer is not ensured.

This problem is overcome by the invention as claimed in claim 1.

A preferred embodiment of the present invention will now be described in detail, which description should be read in conjunction with the accompanying drawings, wherein:

Figure 1 is a lateral side elevation of the orthosis of the present invention shown as mounted to the right leg of the user;

Figure 2 is a front elevation of the same leg showing the orthosis in a different aspect;

Figure 3 is a perspective of the othosis showing the overall construction, and the arrangement for mounting the same, in its operative position, on the user's leg;

Figure 4 is a sectional view of the orthosis, mounted on the leg, taken along section 4—4 of Figure 1, and is intended to show the relative position of the orthosis with respect to the tibia and the calf area of the leg, to illustrate one of the unique features of the invention;

Figure 5 is a side elevation of a typical joint of the present invention, indicating the relative position of the components at various stages of flexion of the knee;

Figure 6 is a side elevation, in partial section, showing the knee joint of Figure 5; and

Figure 7 is an exploded perspective view of a typical joint of the present invention, showing the relative interfitting arrangement of components thereof.

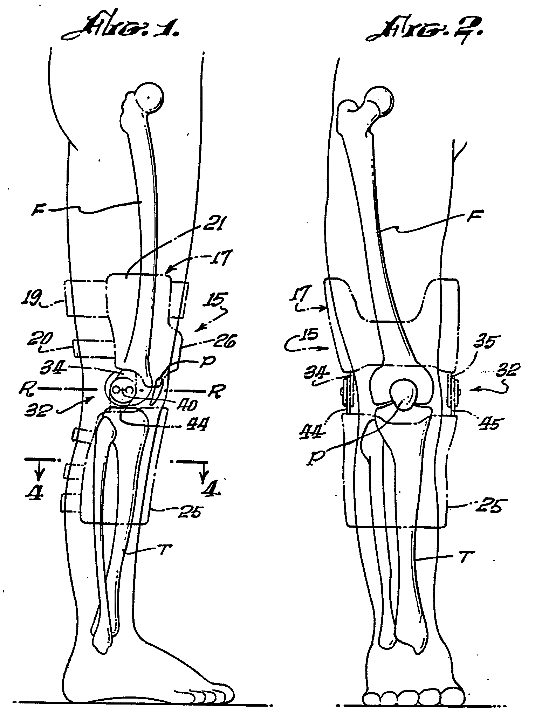

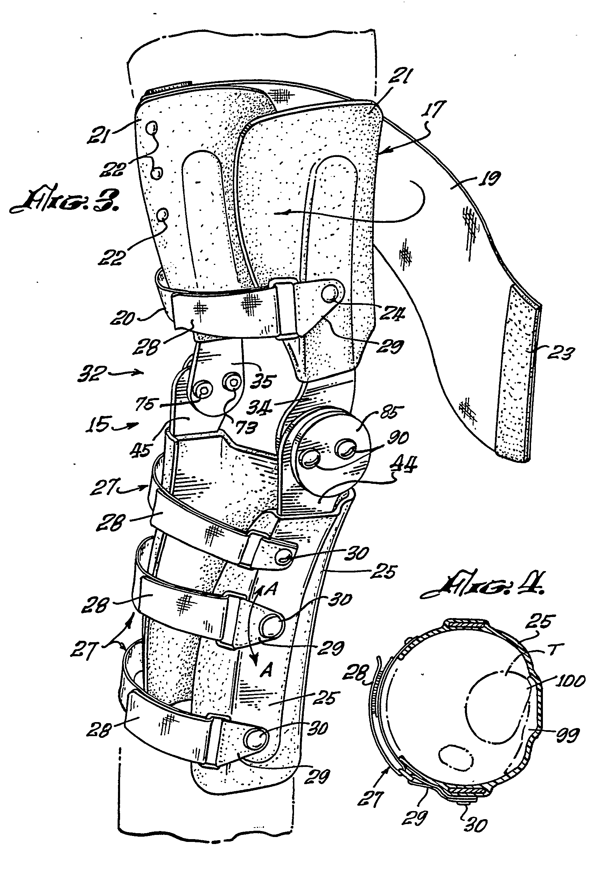

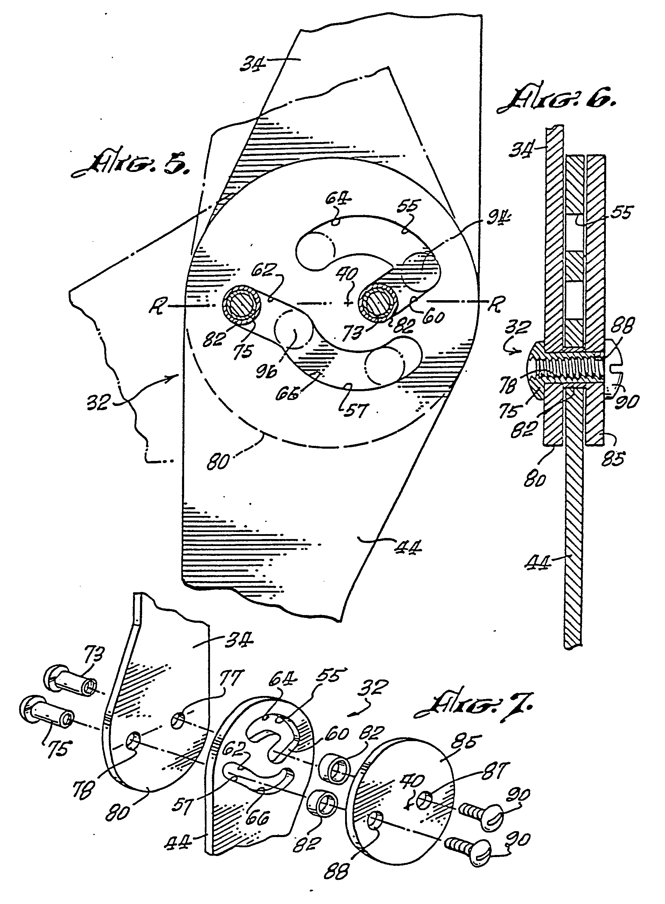

With reference now to the drawings, and more particularly to Figures 1 and 2, a multiaxis controlled motion knee orthosis, or brace, 15 is depicted in its proper mounted position on the right leg of a wearer. So that the fit of the orthosis may be clearly understood, the drawing also depicts the relative positions of the femur F and the tibia T as well as the patella P, forming a typical knee joint of an adult male, indicating the alignment of each with respect to the brace 15, with the wearer standing erect and the right leg in the longitudinally extended position. The brace 15, as may be seen best in Figure 3, includes means for securing the brace to the leg of the wearer, comprising a pair of leg grasping devices in the form of an upper thigh, or femoral, cuff 17 having a major closure means 19 and a minor closure means 20, both of which are constructed so as to encircle the thigh to snugly fit the cuff 17 thereto.

The major closure means encircles the entire upper thigh and is, preferably, of an elastic material, e.g., latex, so as to provide some give as the muscles are flexed, and may be of a latex material, or such other suitable elastic fabric, as desired. The femoral cuff includes a pair of opposed spaced wings 21, adapted to engage the lateral and medial sides of the thigh. The major closure is attached to the medial wing, such as by rivets 22, and a Velcro closure is provided as at 23. The minor closure 20, attaches to the lateral wing at pivot pin 24 and, as illustrated, employs a "D" ring through which the closure is fed and doubled back on itself to provide the desired firmness of fit. Once again, a Velcro closure, or some other closure, is employed.

A calf, or tibial, cuff 25 is disposed beneath the knee joint and partially encircles the calf area, as best seen in Figures 1 and 2. In order to ensure snug fit of the tibial cuff 25, there are provided transverse closure members 27, of which three are shown in longitudinally spaced relation. In order to assure a more precise fit, the femoral and tibial cuffs are formed to specially fit the exact leg upon which the brace is to be worn. This is accomplished by forming a cast about the leg of the intended wearer. The cast is then cut and thereupon serves as a mold to form a model of the leg. Each cuff is then precisely fabricated of a laminate material right on the cast in the proper position. In order to provide optimum strength, both graphite and kevlar filaments are used in a liquid resin, which is then formed about a casting to create the cuffs. Thereafter, the cuffs are lined with some suitably resilient, shock absorbent material such as, for example, sorbathane, and are ready to wear.

The femoral cuff 17, as previously described, is formed with a pair of wings 21 positioned relative to one another by a bridge section 26 spanning the lower part of the thigh above the knee. This construction allows some freedom to allow the larger, upper thigh muscles to expand and contract with loss of control. The area engaged by the tibial cuff includes the greater portion of the calf muscle at the rear of the leg. For that reason, the tibial cuff 25 is more "C" shaped, encircling the majority of the anterior portion of the lower leg, as seen in Figure 4. The cuff is open at the rear of the leg to permit the cuff to be slipped on, and, further, to permit flexing of the calf muscle when the cuff is in place.

The closure members 27 are of a similar construction to the minor closure member 20 secured to the femoral cuff. Thus, each member includes an elastic strap 28 riveted, or otherwise secured, at some point on the medial side of the cuff. A "D" ring is affixed in the lateral plane of the strap by means of a hanger 29 which is shown as attached to the lateral side of the cuff by a fastener 30. The fastener, which may be of any suitable construction, permits limited, essentially longitudinal positioned adjustment by rotation of the hanger 29 along a path A—A so as to permit snug fit over the severe contours of the calf as it flexes during movement. As before, the strap may pass through the "D" ring and close by means of Velcro, or some other suitable closure means.

While the formation and construction of the femoral and tibial cuffs is an important aspect of the invention as a whole, a major feature of the invention rests in the construction of the unique mechanical joint 32, which joins the cuffs to define the brace 15 into an orthosis capable of stabilizing the wearer's knee joint in each of the transverse, coronal and sagittal planes. The detailed construction of the joint 32 of the present invention will now be described with respect to such a joint position at the lateral side of the right knee. It will be appreciated that the medial joint is the mirror image of the lateral joint, and that both will operate in unison as the leg is flexed.

With reference first, primarily, to Figure 3, the femoral cuff 17 is provided with depending, spaced apart, parallel supporting links 34 and 35 respectively. As seen in Figure 3, link 34 is the lateral link and link 35 is the medial link. The links 34 and 35 are readily laminated into the tibial cuff, in a known manner, although it will be appreciated that they may be sewn in, or otherwise fastened, without departure from the invention. The free end of each link 34 and 35, terminates in a bulged, or widened, end respectively. As previously stated, the links are essentially parallel and are disposed on the medial and lateral sides of the thigh, respectively, and depending therefrom to and through a transverse plane R—R shown in Figure 1. The plane R—R is transverse to the longitudinal plane of the tibia F and passes through a center point 40, which represents the center of radius of rotation of the femoral condyle relative to the tibia.

In a like manner, the tibial cuff 25 is provided with upwardly extending, spaced parallel links 44 and 45, respectively, link 44 being on the lateral side, and link 45 being on the medial side of the cuff, as shown. As in the case of the femoral links 34 and 35, the tibial links 44 and 45 extend upwardly, passing through the transverse plane R—R, and each terminates in a widened, or bulged, portion. As in the case of the femoral links, the tibial links may be molded into, sewn, or otherwise attached, to the tibial cuff in some convenient well-known manner. Once again, referring to Figure 3, it will be seen that the lateral femoral and tibial links 34 and 44 are essentially aligned in adjacent planes such that the terminus of each of the links overlaps and is disposed immediately adjacent to one another in a transverse plane, as is the case with the medial links. In each case, the femoral links are disposed slightly inwardly of the tibial links, once again, as best seen in Figure 6.

Overlapping bulged termini portions of the respectively lateral and medial links intersect and lie within the plane R—R to provide the nucleus of the highly unique joint mechanism indicated generally at 32 and illustrated in detail in Figures 5, 6 and 7, respectively. In order to provide optimum stability for an injured knee, and particularly a knee in which the injury involves either the anterior or posterior cruciate ligament, it is necessary to emulate, insofar as reasonably possible, natural, relative movement of the femur and tibia. Most prior art devices appear to have taken for granted that such movement consists of a relative rotation about a single center of rotation, typically in the center of the knee, which is the philosophy resulting in the familiar single pivot brace. Since that is not the natural relative movement of the knee joint, such single pivot braces inherently cause undue stress during flexion of the knee joint, rather than stabilizing, which is the ultimate goal of the present brace. In fact, it has been found that commencing from the leg extended position, the initial movement of the tibia relative to the femur through the first 25° of flexion is an approximate 8 millimeter front to rear slide, followed by rotation as the knee continues to flex through a 125° angle, or for whatever angle may be traversed, as limited by the injury or natural limitations. It has been determined that if the knee is permitted to move in a natural fashion, stress on the cruciate ligaments and the quadriceps muscles, which play a major role in the function on the knee, is so minimal that an injury to the knee is actually permitted to heal more effectively when the user wears the brace of the present invention.

With the foregoing biomechanics in mind, the joint mechanism of the present invention is constructed to permit the knee joint to be constrained to move with the natural glide and rotation of a normal knee, to, thereby, protect the injury and minimize stress by the provision of spaced camming devices, which as illustrated, take the form of longitudinally spaced camming slots 55 and 57, respectively. For purposes of identification, the camming slot 55 will be referred to as the femoral camming slot and camming slot 57 may be referred to as the tibial camming slot. Each slot is formed in a straight line segment 60 and 62, respectively, which adjoins arcuate segments 64 and 66, respectively. It will be noted that the straight line segments 60 and 62 terminate at the end remote from the juncture with the arcuate segments 64 and 66 on the plane R—R. It will also be noted that the arcuate segments are generated about the same radial center which is aligned with the center point 40.

The camming slots are adapted to receive and operate in conjunction with camming follower means, which, as illustrated, comprise screws 73 and 75. As illustrated in Figure 7, a two-part post and screw assembly manufactured by Chicago Screw is shown, although other forms of camming devices could be employed. The camming screws 73 and 75 pass through holes 77 and 78 formed in the terminus 80 of the femoral link (free end). The holes 77 and 78 lie in plane R—R. Bushings 82 are provided, having an inside diameter sufficient to fit over the circumferential surfaces of the posts 73 and 75 in bearing relation with respect thereto, and an outside diameter sufficient to snugly fit in bearing relation within the slots 55 and 57, respectively. A cap, or cover plate, 85 having holes 87 and 88, respectively, permit the receipt of posts 73 and 75, which are then secured by screws 90 to form the joint. The cover plate not only holds the screws, but further serves as a reaction member, sandwiching the bulged terminus of the tibial link between the plate 85 and the bulged terminus 80 of the femoral link, as best seen in Figure 6, such that relative movement of the femoral link relative to the tibial link is strictly controlled by movement of the posts 73 and 75 in the slots 55 and 57, respectively. In Figures 5, 6 and 7, there is shown, by way of example, a lateral joint for the right leg. The medial joint is the mirror image of the lateral joint, so that the relative movement of the lateral and medial links are coordinated and the same.

In order to achieve optimum relative natural movement of the knee joint, which, of course, is a principal feature of the present invention, the interaction of the femoral and tibial surfaces must be taken into account. To this end, while both straight segments 60 and 62 begin with pins 73 and 75, resting in plane R—R, when the leg is extended and essentially straight (see Figure 5), as flexion begins, there is an approximate 8 millimeter lateral movement between the tibial and femoral surfaces, which is permitted as the pins slide along the straight segments 60 and 62. In a very tall person, the slide may be slightly longer, and in a small child, it may be shorter, and the precise length of the slot may be adjusted to meet specific conditions. A range of 6 to 9 millimeters meets most needs. The slide of the femur and tibia, however, does not take place in plane R—R, but, rather, a slight relative tilt is experienced as the leg is flexed through the first 25° of flexion. To accommodate this movement, the straight line segment 60 forms a 50° angle with the plane R—R, whereas, the straight segment 62 forms an angle of 56° 30" (differing from what is depicted in Fig. 5). Thus, as the leg is flexed through the first 25° of flexion, camming posts 73 and 75 respectively, move in a straight line along the straight segments 60 and 62, each at the previously disclosed angle. There is a small tolerance permitted to meet specific needs, but most needs are met within ± 2° of the angles herein specified. At 25°, they intersect the arcuate sections 64 and 66, respectively, at intersections 94 and 96. Beyond 25° of flexion, the natural movement of the knee through further flexion is a relative rotation of the tibia relative to the center point 40 on the femoral condyle. In order that this movement may be emulated with the greatest possible accuracy, arcuate sections 64 and 66 have been formed around center point 40 and it has been found that a radius of .43 inches, or 11 millimeters, provides the desired resultant rotational movement in a typical knee. Approximately 96° 30" is required to provide full knee flexion. It will be appreciated, however, that if a lesser knee flexion is required because of the particular instability found in the user's knee, the arc may traverse a greater or lesser angle without departure from the invention. Indeed, stops may be used to limit flexion simply by inserting a blocking device of known construction and, therefore, not shown, at the appropriate point within the slot 55 and 57 to thereby limit the amount of flexion that the user can experience using the brace.

While in a normal knee there is a slight outward rotation of the tibia relative to the femur upon flexion, such rotation may be pronounced in the case of certain knee injuries resulting in instability. The present invention, in still another of its inventive aspects, limits and constrains such rotation upon flexion of the knee, thereby enhancing the stability thereof. This is accomplished, as best seen in Figure 4, by forming, or molding, the tibial cuff over the anterior boney prominence 100 of the tibia, sometimes referred to as the shin. As seen in Figure 4, the cuff has been molded, or formed, about that prominence, such that any tendency of the tibia to rotate about its longitudinal axis upon flexion, is resisted by the cuff. The structure of the cuffs and links is of such integrity that not only is rotation controlled within the cuff, but when the leg is flexed, the wings 21 of the femoral cuff, work with the tibial cuff by acting against the wearer's thigh through the links to resist any rotation of the brace, thereby totally inhibiting undesirable rotation. In order to avoid irritation by rubbing of the tibia against this tightly molded section of the cuff, a spongy material, such as sorbathane, may be provided in this area about the interior of the cuff, at or about the location 99, or about the entire area which contacts the skin of the wearer if it is so desired. Strong closure members 27, 28 and 29, of course, permit the tibial cuff to be held in position firmly against the shin, while at the same time, minimizing the discomfort which might otherwise be experienced by the wearer.

**Claims**

1. An appliance (15) for stabilizing a knee joint in sagittal, coronal and transverse planes comprising

leg grasping means (17, 25) for clasping the appliance to the wearer's leg above and below the knee,

means defining a mechanical joint (32) at the medial and lateral sides of the knee, each comprising

a pair of opposed depending femoral links (34, 35), each link terminating in an end portion (80),

a pair of opposed upwardly extending tibial links (44, 45) terminating in an end portion, said end portion of said tibial links (44, 45) being disposed in an overlapping relation to said end portion of said femoral links (34, 35),

means interconnecting each said end portion (80) of said femoral links (34, 35) to a respective said end portion of said tibial links (44, 45), said interconnecting means being disposed at lateral and medial sides of the knee and allowing said femoral and tibial links to perform relative sliding and rotational movements, characterized by

each said interconnecting means comprising cam means,

each said cam means comprising a cam pin follower (73, 82; 75, 82) and a camming slot means (55, 57), each said cam pin follower means being fixedly positioned relative to one of said pair of femoral links (34, 35) and relatively displaceable to a link of said other pair of links (44, 45) within a respective camming slot means (55, 57), said cam pin follower (73, 82; 75, 82) and camming slot means (55, 57) forming a means for constraining the tibia to slide rearwardly relative to the femur for a predetermined distance during an initial range of flexion from a straight leg portion and beyond said initial range of flexion to, thereafter, rotate relative thereto in a predetermined arcuate path.

2. An appliance as set forth in claim 1, wherein each said camming slot means comprises a pair of camming slots (55, 57), spaced from each other, in a direction towards femur and tibia, and each said cam pin follower means comprises a pair of pins (73, 82; 75, 82), each passing through one of said tibial and femoral camming slots.

3. An appliance as set forth in claim 2, wherein each said camming slot (55, 57) comprises a straight segment (60, 62) adjoining an arcuate segment (64, 66).

4. An appliance as set forth in claim 2 or 3, wherein each said cam pin follower means comprises a camming screw (73, 75) extending outwardly from said end portion (80) of said femoral link, and passing through one of said slots (55, 57), means defining a cap (85), said cap means disposed adjacent to and outwardly of said end portion of said tibial link (44, 45), said cap means receiving said camming screw (73, 75) in holding engagement.

5. An appliance as set forth in claim 4, wherein bushing means (82) is mounted on said camming screw (73, 75) in bearing relation and has a circumferential surface in bearing engagement with said camming slot (55, 57).

6. An appliance as set forth in any one of the claims 2 to 5, wherein said cam pin follower means are disposed in a horizontal plane (R—R) intersecting a center point (40) representing the center of radius of the femoral condyle of the wearer, the pair of camming slots (55, 57) comprising upper and lower camming slots which extend from said plane in directions above and below said plane.

7. An appliance as set forth in any one of the claims 3 to 6, wherein the arcuate segment (64, 66) of each said camming slot (55, 57) is of equal radius circumscribed about a centre point (40) representing the radius of the femoral condyle of the wearer.

8. An appliance as set forth in claim 3 or 7, wherein the straight segment (62) of one (57) of said camming slots (55, 57) extends from the arcuate segment (66) toward the posterior of the joint, and the straight segment (60) of the other camming slot (55) extends towards the anterior of the joint.

9. An appliance as set forth in claim 3, wherein said straight segment (60, 62) is 8 millimeters ± 2 millimeters in length, and wherein the longitudinal axis of said straight segment (60) of said femoral camming slot (55) requires a 50° ± 2° angle with respect to said transverse plane (R—R), and the longitudinal axis of said straight segment (62) of said tibial camming slot (57) defining a 56° 30' ± 2° angle with respect to said transverse plane, and the radius of each said arcuate segment (64, 66) is about 11 millimeters.

10. An appliance as set forth in any one of the preceding claims, wherein the initial range of flexion is about 25°.

11. An appliance as set forth in any one of the preceding claims, wherein the leg grasping means comprises a femoral cuff (15) formed to fit snugly about a portion of the thigh, closure means (19, 20) attached to said femoral cuff (15) for securing said femoral cuff to a portion of a leg above the knee joint, a tibial cuff (25) formed to fit snugly about the anterior portion of the tibia and calf of the leg below the knee joint, closure means (27, 28, 29) attached to said tibial cuff (25) for securing said tibial cuff in place, said tibial cuff (25) being molded about the boney prominence (100) of the tibia so as to prevent rotation of said tibia on its longitudinal axis.

12. An appliance set forth in claim 11, wherein said closure means (27, 28, 29) attached to said tibial cuff (25) comprises at least three spaced elastic straps (28) being adjustable to vary tightness of said tibial cuff (25) about the leg while holding said cuff in proper position, and means (30) permitting limited longitudinal movement of said elastic straps (28).

13. An appliance as set forth in claim 11 or 12, wherein said femoral cuff (17) comprises spaced wings (21) positioned to engage the lateral and medial area of the wearer's thigh, and means (19, 20) defining a bridge and joining said wings to form a rigid unit.

## Patentansprüche

1. Vorrichtung (15) zum Stabilisieren eines Kniegelenks in sagittalen, coronalen und transversalen Ebenen, mit Beinfaßmitteln (17, 25) zum Festmachen der Vorrichtung am Bein des Trägers ober- und unterhalb des Knies, ein mechanisches Gelenk (32) definierenden Mitteln an der medialen und lateralen Seite des Knies, jeweils umfassend ein Paar von gegenüberliegenden sich nach unten erstreckenden femoralen Gliedern (34, 35), von denen jedes Glied in einem Endabschnitt (80) endet, ein Paar von gegenüberliegenden sich nach oben erstreckenden tibialen Gliedern (44, 45), die in einem Endabschnitt enden, wobei der Endabschnitt der tibialen Glieder (44, 45) in Überlappbeziehung zu dem Endabschnitt der femoralen Glieder (34, 35) angeordnet ist, Mittel, welche jeden Endabschnitt (80) der femoralen Glieder (34, 35) mit einem entsprechenden Endabschnitt der tibialen Glieder (44, 45) verbinden, wobei die Verbindungsmittel an der lateralen und medialen Seite des Knies angeordnet sind und eine relative Verschiebe-und Drehbewegung der femoralen und tibialen Glieder gestatten, dadurch gekennzeichnet, daß jedes der Verbindungsmittel Nockenmittel umfaßt, jedes der Nockenmittel einen Nockenfolgestift (73, 82; 75, 82) und Nockenschlitzmittel (55, 57) umfaßt, wobei jeder Nockenfolgestift fest bezüglich einem des Paares von femoralen Gliedern (34, 35) angeordnet und bezüglich einem Glied des anderen Paares von Gliedern (44, 45) innerhalb zugehöriger Nockenschlitzmittel (55, 57) verschiebbar ist, wobei der Nockenfolgestift (73, 82; 75, 82) und die Nockenschlitzmittel (55, 57) Mittel bilden, welche zwangsweise die Tibia bezüglich des Femurs über eine bestimmte Strecke während eines Anfangsbeugebereichs aus einer gestreckten Beinlage heraus nach hinten gleiten lassen und jenseits des Anfangsbeugebereichs dann relativ zu ihm in einem bestimmten bogenförmigen Weg drehen lassen.

2. Vorrichtung nach Anspruch 1, bei welcher jedes der Nockenschlitzmittel ein Paar von Nockenschlitzen (55, 57) umfaßt, die voneinander in Richtung nach Femur und Tibia getrennt sind, und jedes der Nockenstiftfolgemittel ein Paar von Stiften (73, 82; 75, 82) umfaßt, von denen jeder einen der tibialen und femoralen Nockenschlitze durchsetzt.

3. Vorrichtung nach Anspruch 2, bei welcher jeder der Nockenschlitze (55, 57) ein gerades Segment (60, 62) angrenzend an ein gekrümmtes Segment (64, 66) umfaßt.

4. Vorrichtung nach Anspruch 2 oder 3, bei welcher jedes der Nockenfolgestiftmittel eine Nockenschraube (73, 75), die sich von dem Endabschnitt (80) des femoralen Gliedes nach außen erstreckt und einen der Schlitze (55, 57) durchsetzt, eine Kappe (85) definierende Mittel umfaßt, wobei die Kappenmittel benachbart und außenseitig des Endabschnitts des tibialen Gliedes (44, 45) angeordnet sind, wobei die Kappenmittel die Nockenschraube (73, 75) in haltendem Eingriff aufnehmen.

5. Vorrichtung nach Anspruch 4, bei welcher Hülsenmittel (82) auf der Nockenschraube (73, 75) in Lagerbeziehung angebracht sind und eine Umfangsoberfläche aufweisen, die in Lagereingriff mit dem Nockenschlitz (55, 57) ist.

6. Vorrichtung nach irgendeinem der Ansprüche 2 bis 5, bei welcher die Nockenfolgestiftmittel in einer horizontalen Ebene (R—R) angeordnet sind, welche durch einen Mittelpunkt (40) geht, der die Radiusmitte des femoralen Condylus des Trägers darstellt, wobei das Paar von Nockenschlitzen (55, 57) obere und untere Nockenschlitze umfaßt, welche sich aus der Ebene in Richtungen oberhalb und unterhalb der Ebene erstrecken.

7. Vorrichtung nach irgendeinem der Ansprüche 3 bis 6, bei welcher das gekrümmte Segment (64, 66) eines jeden Nockenschlitzes (55, 57) von um einen Mittelpunkt (40) umschriebenem, gleichem Radius ist, welcher den Radius des femoralen Condylus des Trägers darstellt.

8. Vorrichtung nach Anspruch 3 oder 7, bei welcher das gerade Segment (62) eines (57) der Nockenschlitze (55, 57) sich vom gekrümmten Segment (66) zum hinteren Teil des Gelenks hin erstreckt und das gerade Segment (60) des anderen Nockenschlitzes (55) sich zum Vorderteil des Gelenks hin erstreckt.

9. Vorrichtung nach Anspruch 3, bei welcher das gerade Segment (60, 62) eine Länge von 8 mm ± 2 mm hat, und bei welcher die Längsachse des geraden Segments (60) des femoralen Nokkenschlitzes (55) einen 50° ± 2° Winkel in Bezug auf die transversale Ebene (R—R) erfordert und die Längsachse des geraden Segments (62) des tibialen Nockenschlitzes (57) einen 56° 30' ± 2° Winkel in Bezug auf die transversalen Ebene definiert, und der Radius eines jeden gekrümmten Segments (64, 66) ungefähr 11 mm beträgt.

10. Vorrichtung nach irgendeinem der vorstehenden Ansprüche, bei welcher der Anfangsbereich der Beugung ungefähr 25° beträgt.

11. Vorrichtung nach irgendeinem der vorstehenden Ansprüche, bei welcher die Beinfaßmittel eine femorale Manschette (15), die so ausgebildet ist, daß sie eng um einen Abschnitt des Oberschenkels anliegt, an der femoralen Manschette (15) angebrachte Verschlußmittel (19, 20) zum Befestigen der femoralen Manschette an einem Teil eines Beines oberhalb des Kniegelenks, eine tibiale Manschette (25), die eng um den vorderen Teil der Tibia und Wade des Beins unterhalb des Kniegelenks anliegt, an der tibialen Manschette (25) angebrachte Verschlußmittel (27, 28, 29) zum Instellunghalten der tibialen Manschette umfassen, wobei die tibiale Manschette (25) um die knochige Hervorragung (100) der Tibia herum geformt ist, um so eine Drehung der Tibia auf ihrer Längsachse zu verhindern.

12. Vorrichtung nach Anspruch 11, bei welcher die an der tibialen Manschette (25) angebrachten Verschlußmittel (27, 28, 29) wenigstens drei im Abstand liegende elastische Gurte (28), die für eine Veränderung der Enge, mit der die tibiale Manschette (25) um das Bein herum anliegt, einstellbar sind und dabei die Manschette in richtiger Lage halten, und Mittel (30), die eine begrenzte Längsbewegung der elastischen Gurte (28) gestatten, umfassen.

13. Vorrichtung nach Anspruch 11 oder 12, bei welcher die femorale Manschette (17) im Abstand liegende Flügel (21), die so angeordnet sind, daß sie am seitlichen und medialen Bereich des Oberschenkels des Trägers angreifen, und Mittel (19, 20), die eine Brücke bestimmen und die Flügel zu einer starren Einheit verbinden, umfaßt.

**Revendications**

1. Dispositif (15) pour stabiliser une articulation du genou dans les plans sagittal, coronal et transversal comprenant un organe de saisie de la jambe (17, 25) pour plaquer le dispositif sur la jambe de l'utilisateur au-dessus et en dessous du genou, un organe définissant une articulation mécanique (32) aux côtés médial et latéral du genou, chacun comprenant une paire de'liens fémoraux dépendants opposés (34, 35), chaque lien se terminant dans une partie finale (80), une paire de liens tibiaux s'étendant vers le haut de manière opposée (44, 45) se terminant dans une partie finale, ladite partie finale desdists liens tibiaux (44, 45) étant disposée dans une relation de recouvrement avec ladite partie finale desdits liens fémoraux (34, 35), un organe interconnectant chacune desdites parties finales (80) desdits liens fémoraux (34, 35) à une desdites parties finales respectives desdits liens tibiaux (44, 45), ledit organe d'interconnection étant disposé aux côtés latéral et médial du genou et permettant auxdits liens fémoral et tibial d'effectuer des mouvements relatifs de glissement et de rotation, caractérisé en ce que chacun desdits moyens d'interconnection comprend desorganes de guidage, chacun desdits organes de guidage comprenant un organe suivant la broche de guidage (73, 82; 75, 82) et un organe de rainure de guidage (55, 57), chacun desdits éléments suivant la broche de guidage étant disposé de façon fixe relativement à l'une desdites paires de liens fémoraux (34, 35) et relativement déplaçable vers un lien de ladite autre paire de liens (44, 45) dans un organe de rainure de guidage respectif (55, 57), ledit organe suivant la broche de guidage (73, 82; 75, 82) et ledit organe de rainure de guidage (55, 57) formant un moyen pour contraindre le tibia à glisser vers l'arrière relativement au fémur, sur une distance prédéterminée pendant un intervalle initial de flexion à partir d'une partie de jambe droite et au-delà dudit intervalle de flexion pour ensuite tourner relativement à lui selon une trajectoire arquée prédéterminée.

2. Dispositif selon la revendication 1, dans lequel chacun desdits organes de rainures de guidage comprend une paire de rainures de guidage (55, 57) espacées l'une de l'autre dans une direction allant vers le fémur et le tibia, et chacun desdits organes suivant la broche de guidage comprend une paire de broches (73, 82; 75, 82), passant chacune à travers l'une desdites rainures de guidage tibiale et fémorale.

3. Dispositif selon la revendication 2, dans lequel chacune desdites rainures de guidage (55, 57) comprend un segment droit (60, 62) joignant un segment arqué (64, 66).

4. Dispositif selon la revendication 2 ou 3, dans lequel chacun desdits organes suivant la broche de guidage comprend une vis de guidage (73, 75) s'étendant vers l'extérieur à partir de ladite partie finale (80) dudit lien fémoral, et passant à travers l'une desdites rainures (55, 57), un organe définissant un couvercle (85), ledit organe de couverture disposé de façon adjacente et à l'extérieur de ladite partie finale dudit lien tibial (44, 45), ledit organe de couverture recevant ladite vis de guidage (73, 75) dans un engagement de tenue.

5. Dispositif selon la revendication 4, dans lequel un organe de coussinet (82) est monté sur ladite vis de guidage (73, 75) dans une relation de coussinet et a une surface circonférentielle en engagement de coussinet avec ladite rainure de guidage (55, 57).

6. Dispositif selon l'une quelconque des revendications 2 à 5, dans lequel lesdits organes suivant la broche de guidage sont disposés dans un plan horizontal R—R coupant un point central (40) représentant le centre du rayon du condyle fémoral de l'utilisateur, la paire de rainures de guidage (55, 57) comprenant des fentes de guidage supérieure et inférieure s'étendant à partir dudit plan dans des directions allant au-dessus et audessous dudit plan.

7. Dispositif selon l'une quelconque des revendications 3 à 6, dans lequel le segment arqué (64, 66) de chacune desdites fentes de guidage (55, 57) est de rayon égal circonscrit autour d'un point central (40) représentant le rayon du condyle fémoral de l'utilisateur.

8. Dispositif selon la revendication 3 ou 7, dans lequel le segment central (62) d'une (57) desdites fentes de guidage (55, 57) s'étend à partir du segment arqué (66) vers l'arrière de l'articulation, et le segment droit (60) de l'autre fente de guidage (55) s'étend vers la partie antérieure de l'articulation.

9. Dispositif selon la revendication 3, dans lequel ledit segment droit (60, 62) a 8 mm ± 2 mm de longueur, et où l'axe longitudinal dudit segment droit (60) de ladite fente de guidage fémorale (55) nécessite unangle de 50° ± 2° par rapport audit plan transversal R—R, et l'axe longitudinal dudit segment droit (62) de ladite fente de guidage tibiale (57) définissant un angle de 56°30' ± 2° par rapport audit plan transversal, et le rayon de chacun desdits segments arqués, (64, 66) est d'environ 11 mm.

10. Dispositif selon l'une quelconque des revendications précédentes, dans lequel l'intervalle de flexion initial est d'environ 25°.

11. Dispositif selon l'une quelconque des revendications précédentes dans lequel le moyen pour saisir la jambe comprend une manchette fémorale (15) formée de manière à pouvoir s'adapter étroitement autour d'une partie de la cuisse, un organe de fermeture (19, 20) attaché à ladite manchette fémorale (15) pour fixer ladite manchette fémorale à une partie de jambe audessus de l'articulation du genou, une manchette tibiale (25) formée pour s'adapter étroitement autour de la partie antérieure du tibia et du mollet de la jambe en dessous de l'articulation du genou, un organe de fermeture (28, 29) attaché à ladite manchette tibiale (25) pour fixer ladite manchette tibiale en place, ladite manchette tibiale (25) étant moulée autour de la proéminence osseuse (100) du tibia de manière à empêcher la rotation dudit tibia sur son axe longitudinal.

12. Dispositif selon la revendication 11, dans lequel ledit organe de fermeture (27, 28, 29) attaché à ladite manchette tibiale (25) comprend au moins trois sangles élastiques espacées (28) pouvant être ajustées afin de faire varier l'étroitesse de ladite manchette tibiale (25) autour de la jambe tout en maintenant ladite manchette en position appropriée et un organe (30) permettant un mouvement longitudinal limité desdites sangles élastiques (28).

13. Dispositif selon la revendication 11 ou 12, dans lequel ladite manchette fémorale (17) comprend des ailes espacées (21) positionnées de manière à engager la zone latérale et médiale de la cuisse de l'utilisateur, et un organe (19, 20) définissant un pont et réunissant lesdites ailes pour former une unité rigide.

EP  0 173 161  B1

Fig. 1.

Fig. 2.

1

*Fig. 3.*

*Fig. 4.*

2

EP 0 173 161 B1

Fig. 5.

Fig. 6.

Fig. 7.